# EUROPEAN PATENT APPLICATION

(11) **EP 4 454 636 A1**
(43) Date of publication of application: **30.10.2024**
(21) Application number: 22909831.4
(22) Date of filing: 14.12.2022
(51) Int. Cl.: A61K 9/00, A61K 47/34

(54) **CONTINUOUS DELIVERY PREPARATION CAPABLE OF BEING STABLY RELEASED AND PREPARATION METHOD THEREFOR**

(30) Priority: 24.12.2021 CN 202111598937
(71) Applicant: Sichuan Kelun Pharmaceutical Research Institute Co., Ltd., Chengdu, Sichuan 611138 (CN); Hunan Kelun Pharmaceutical Research Co., Ltd., Yueyang, Hunan 414000 (CN)
(72) Inventor: SU, Zhengxing, Chengdu, Sichuan 611138 (CN); YANG, Yifan, Chengdu, Sichuan 611138 (CN); ZHAO, Jinlong, Chengdu, Sichuan 611138 (CN); XU, Wei, Chengdu, Sichuan 611138 (CN); YANG, Xueyuan, Chengdu, Sichuan 611138 (CN); ZHAO, Dong, Chengdu, Sichuan 611138 (CN); LIU, Sichuan, Chengdu, Sichuan 611138 (CN)
(74) Representative: Cabinet Laurent & Charras
(86) International application number: PCT/CN2022/138871
(87) International publication number: WO 2023/116517

(57) **Abstract**

A continuous delivery preparation capable of being stably released and a preparation method therefor. The preparation comprises an active pharmaceutical ingredient and a gel carrier, and the gel carrier comprises a biodegradable polymer, an organic solvent, a hydrophobic additive, and an optional hydrophilic gel matrix material. Compared with an in-situ gel prepared by means of a conventional Atrigel technology, the delivery preparation has the effect of slowing down in vitro and vivo burst release of the drug after a small amount of the hydrophobic additive and the optional hydrophilic gel matrix material are added, and the in-vivo blood drug concentration can be maintained for more than one week in a safe and effective range. The preparation method of the preparation is simple.

## Description

### CROSS-REFERENCE TO RELATED APPLICATION

The present disclosure claims priority of Chinese Patent Application No. 202111598937.X, filed on December 24, 2021, entitled "CONTINUOUS DELIVERY PREPARATION CAPABLE OF BEING STABLY RELEASED AND PREPARATION METHOD THEREFOR", which is hereby incorporated by reference in its entirety.

### TECHNICAL FIELD

The present disclosure belongs to the technical field of pharmaceutical formulations, and specifically relates to a continuous delivery preparation capable of being stably released, and a preparation method for the delivery preparation.

### BACKGROUND

In situ forming implant (ISFI) is a novel drug delivery system composed of a solution or semi-solid mixture of biodegradable polymer, which is capable of being solidified/gelated at the injection site to form a depot. Depending on the composition of formulation, ISFIs are primarily classified by different mechanisms of formation such as in situ precipitation, organic gelation, or polymer crosslinking. Of these, Atrigel^{®} system, based on solvent diffusion-induced in situ precipitation, is currently market-oriented and the most applied technology with several commercial products having been put on the market, and is widely used in the fields such as treatment of periodontitis (Atridox^{®}), advanced pancreatic cancer (Eligard^{®}), opioid use disorder (Sublocade^{®}), and schizophrenia (Perseris^{®}).

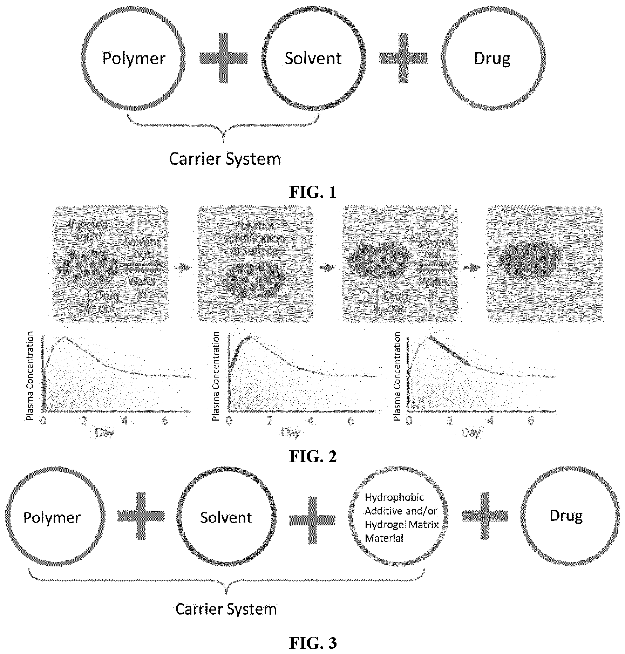

Dunn *et al.* first put forward the concept related to the Atrigel^{®} technology in 1987 (as shown in FIG. 1). This technology is intended mainly to mix a biodegradable polymer carrier (lactide/glycolide copolymer (poly(lactic-co-glycolic), PLGA) or polylactide (polylactic acid, PLA)) with an organic solvent (N-methyl pyrrolidone NMP) till dissolution to form a carrier system, and then dissolve or disperse a small molecular, polypeptide, or macromolecular drug therein to form an injectable solution or suspension. After injection of the preparation, the solvent diffuses to cause phase separation and penetrates into the surrounding tissue fluid, while the polymer precipitates at the injection site to form a semi-solid or solid drug depot, thereby achieving an effect of long-acting release. Due to characteristics of the formulation, the Atrigel^{®} in situ gels boast the advantages of simple preparation process, facile injection method, easy mass production, etc., as compared to conventional solid implants and microspheres.

As a result of the formation principle of formulation, the Atrigel^{®} in situ gel preparation tends to exhibit a large burst release. The *in vivo* release process of a drug in the in situ gel can be divided into three main stages: a burst release stage, a diffusion stage, and a polymer degradation stage (as shown in FIG. 2). The burst release stage mainly means that when the preparation is injected, the organic solvent is brought into contact with tissue fluid to cause phase separation, thereby resulting in a rapid release of the drug. Numerous studies have shown that the ratio of drug release at this stage may be between 8% and 95%. For central nervous system diseases such as Parkinson's disease, Alzheimer's disease, and schizophrenia, the drug for treatment usually has a narrow safety window, thus the fluctuation range of the plasma concentration needs to be strictly controlled. However, the burst release of the drug often leads to a rapid increase of drug exposure *in vivo,* which in turn causes serious toxic and side effects.

Therefore, how to solve the burst release of drug in Atrigel^{®} and obtain a preparation capable of being released continuously and steadily for a week, a month, or even longer is currently a major problem that needs to be addressed urgently.

### SUMMARY

### Technical Problem

In view of the problem about a larger release amount of drug in the Atrigel^{®} in situ gel preparation at the burst release stage *in vivo,* the present disclosure provides a continuous delivery preparation capable of being stably released, and addresses the problem about a larger initial burst release of conventional in situ gel (Atrigel technology).

### Solution to Problem

[1]. A delivery preparation, comprising an active pharmaceutical ingredient and a gel carrier, wherein the gel carrier comprises a biodegradable polymer, an organic solvent, a hydrophobic additive, and optionally a hydrophilic gel matrix.
[2]. The delivery preparation according to [1], wherein the hydrophobic additive is one or more selected from the group consisting of ethyl acetate, medium-chain triglyceride, glyceryl triacetate, glyceryl tricaprylate, benzyl benzoate, and benzyl alcohol; preferably, the hydrophobic additive is one or more selected from the group consisting of benzyl benzoate, glyceryl triacetate, and glyceryl tricaprylate.
[3]. The delivery preparation according to [1] or [2], wherein the hydrophobic additive is 1% to 50% of the total mass of the gel carrier, preferably 5% to 30% of the total mass of the gel carri er.
[4]. The delivery preparation according to any one of [1] to [3], wherein the hydrophilic gel matrix is one or more selected from the group consisting of poloxamer, carbomer, polyvinylpyrrolidone, hydroxypropyl methyl cellulose, and sodium carboxymethyl cellulose; preferably, the hydrophilic gel matrix is one or more selected from the group consisting of poloxamer 188, carbomer, and polyvinylpyrrolidone.
[5]. The delivery preparation according to any one of [1] to [4], wherein the hydrophilic gel matrix, if present, is 0.5% to 15% of the total mass of the gel carrier, preferably 1% to 5% of the total mass of the gel carrier.
[6]. The delivery preparation according to any one of [1] to [5], wherein the biodegradable polymer is a polyester or a polyester copolymer; preferably, the biodegradable polymer is polylactide or lactide/glycolide copolymer; preferably, the biodegradable polymer is lactide/glycolide copolymer; more preferably, the molar ratio of lactide to glycolide in the lactide/glycolide copolymer is 50:50 to 95:5; and/or the biodegradable polymer has a molecular weight of 5000 to 70000 Da; and/or the biodegradable polymer is 20% to 50% of the total mass of the gel carrier.
[7]. The delivery preparation according to any one of [1] to [6], wherein the organic solvent is N-methyl pyrrolidone and/or dimethyl sulfoxide; and/or the mass ratio of the organic solvent to the hydrophobic additive is 1: 1 to 9: 1.
[8]. The delivery preparation according to any one of [1] to [7], wherein the active pharmaceutical ingredient is 0.5% to 30% of the total mass of the delivery preparation; preferably, the active pharmaceutical ingredient is 3% to 20% of the total mass of the delivery preparation.
[9]. A preparation method for the delivery preparation according to any one of [1] to [8], comprising the following steps: weighing prescribed doses of a biodegradable polymer, an organic solvent, a hydrophobic additive, and optionally a hydrophilic gel matrix, and mixing to homogeneity, adding an active pharmaceutical ingredient, and mixing to homogeneity to obtain the delivery preparation.
[10]. The preparation method according to [9], wherein the active pharmaceutical ingredient is added just before administration of the delivery preparation.
[11]. The preparation method according to [9], wherein the active pharmaceutical ingredient is added immediately after the biodegradable polymer, the organic solvent, the hydrophobic additive, and optionally the hydrophilic gel matrix are mixed to homogeneity.
[12]. A preparation method for the delivery preparation according to any one of [1] to [8], comprising the following steps: weighing prescribed doses of a biodegradable polymer, an active pharmaceutical ingredient, an organic solvent, a hydrophobic additive, and optionally a hydrophilic gel matrix, and mixing to homogeneity to obtain the delivery preparation.

### Advantageous Effects of the Invention

The present disclosure provides a continuous delivery preparation capable of being stably released. As compared to the in situ gel prepared by the conventional Atrigel technology, by means of adding a small amount of a hydrophobic additive and optionally a hydrophilic gel matrix, the above preparation achieves significant results in retarding the burst release of drug *in vivo* and *in vitro* and maintaining the *in vivo* plasma concentration in a safe and effective range over more than one week. At the same time, the preparation method for the above preparation is simple and suitable for large-scale production. Moreover, the finished delivery preparation is convenient in use.

### BRIEF DESCRIPTION OF THE DRAWINGS

FIG. 1 shows a schematic diagram for the composition of an Atrigel^{®} product.
FIG. 2 shows a schematic diagram for the release mechanism of an Atrigel^{®} product.
FIG. 3 shows a schematic diagram for the composition of a delivery preparation of the present disclosure.
FIG. 4 shows plasma concentration vs. time curves over 504 h for the samples of Examples 5, 10, 11, and 12 in rats.
FIG. 5 shows plasma concentration vs. time curves over 240 h for the samples of Examples 13, 14, and 15 in rats.
FIG. 6 shows a plasma concentration vs. time curve over 312 h for the sample of Example 28 in rats.
FIG. 7 shows plasma concentration vs. time curves over 504 h for the samples of Examples 1, 3, and 5 in rats.
FIG. 8 shows plasma concentration vs. time curves over 504 h for the samples of Examples 10, 11, and 12 in rats.
FIG. 9 shows plasma concentration vs. time curves over 720 h for the samples of Examples 13 and 14 in rats.
FIG. 10 shows plasma concentration vs. time curves over 720 h for the samples of Examples 15, 16, 29, and 30 in rats.

### DETAILED DESCRIPTION

Embodiments of the present disclosure will be described below, but the present disclosure is not limited thereto. The present disclosure is not limited to the elements described below. Various alterations may be made within the claimed scope. Moreover, embodiments or examples obtained by appropriately combining the technical means disclosed respectively in different embodiments or examples are also encompassed in the technical scope of the present disclosure.

Unless otherwise defined, the technical and scientific terms used herein have the same meanings as typically understood by one of ordinary skill in the art to which the present disclosure belongs.

The numerical range represented by "numerical value A to numerical value B" or "numerical value A - numerical value B" used herein refers to the range including the endpoint values A and B.

The term "may" used herein involves both the meaning of performing some treatment and the meaning of not performing some treatment. In the present specification, the term "optional" or "optionally" means that the event or situation described subsequently may or may not occur, and the description includes the situation where the event occurs and the situation where the event does not occur.

The term "a" or "an" or "the" used herein may refer to "one", or refer to "one or more", "at least one", and "one or more than one".

The term "about" used herein may indicate that a value includes the standard deviation of an error caused by the device or method used to measure the value. The numerical ranges and parameters used to define the present disclosure are all approximate values, and the relevant values in the specific examples have been presented as accurately as possible. Any numerical value, however, inherently and inevitably contains a standard deviation resulting from the aforementioned testing devices or methods. Therefore, unless otherwise expressly stated, it shall be appreciated that all ranges, quantities, values, and percentages used herein have already been modified by "about". The term "about" used herein generally means that the actual value is within ±10%, ±5%, ±1% or ±0.5% of a specific value or range.

The parameters for PLGA used herein are explained as follows: taking PLGA (7525 DLG 2A) as an example, "7525" means that the molar ratio of lactide to glycolide is 75:25; "DLG" means lactide/glycolide copolymer, and if represented as "DL", it means polylactide; "2" acts as a sign for intrinsic viscosity, where 1 means that the intrinsic viscosity is 0.05 to 0.15 dL/g, 1.5 means that the intrinsic viscosity is 0.10 to 0.20 dL/g, 2 means that the intrinsic viscosity is 0.15 to 0.25 dL/g, 2.5 means that the intrinsic viscosity is 0.20 to 0.30 dL/g, 3 means that the intrinsic viscosity is 0.25 to 0.35 dL/g, 3.5 means that the intrinsic viscosity is 0.30 to 0.40 dL/g, 4 means that the intrinsic viscosity is 0.35 to 0.45 dL/g, 4.5 means that the intrinsic viscosity is 0.40 to 0.50 dL/g, 5 means that the intrinsic viscosity is 0.45 to 0.55 dL/g, 6 means that the intrinsic viscosity is 0.50 to 0.70 dL/g, 7 means that the intrinsic viscosity is 0.60 to 0.80 dL/g, 8 means that the intrinsic viscosity is 0.70 to 0.90 dL/g, and 9 means that the intrinsic viscosity is 0.80 to 1.00 dL/g; the PLGA having a molecular weight of 100000 Da has an intrinsic viscosity of 1 dL/g; and "A" means that the end group is an acid, and if represented as "E", it means that the end group is an ester.

### <First Aspect>

In the first aspect, the present disclosure provides a gel carrier, comprising a biodegradable polymer, an organic solvent, a hydrophobic additive, and optionally a hydrophilic gel matrix.

### [Gel Carrier]

In the present disclosure, "gel carrier" refers to a substance or a composition of multiple substances used for encapsulating, delivering, and/or releasing a bio-activator or an active pharmaceutical ingredient, for example, including a biodegradable polymer and an organic solvent.

In some specific embodiments, the gel carrier of the present disclosure comprises a biodegradable polymer, an organic solvent, a hydrophobic additive, and optionally a hydrophilic gel matrix. In some more specific embodiments, the gel carrier of the present disclosure comprises a biodegradable polymer, an organic solvent, and a hydrophobic additive. In other more specific embodiments, the gel carrier of the present disclosure comprises a biodegradable polymer, an organic solvent, a hydrophobic additive, and a hydrophilic gel matrix.

### [Biodegradable Polymer]

In the present disclosure, "biodegradable polymer", "bioabsorbable polymer", and "absorbable polymer" may be used interchangeably and refer to polymers that may break down by chemical or physical methods when interacting with a physiological environment, for example, the polymers are eroded, decomposed, or dissolved at the implantation site in a subject over a period of time, e.g. within several days, several weeks, or several months. Biodegradable polymers serve a temporary function in a subject, e.g. delivering bio-activators such as drugs. Biodegradable polymers may be degraded into fragments and metabolized or excreted by the host. The specific type of the biodegradable polymer is not particularly limited in the present disclosure and may be determined by a person skilled in the art as actually required.

In the present disclosure, the biodegradable polymer may be a polyester that refers to a polymer in which all or substantially all of the repeating units are linked together by ester groups. The polyester may be formed by subjecting a monomer having carboxyl group and a monomer having hydroxyl group to reaction in order to form ester group. The polyester may be formed by the ring opening polymerization of a cyclic ester monomer. The polyester may be formed by a monomer such as glycolide, lactide, ε-caprolactone, or p-dioxanone. In some specific embodiments, the biodegradable polymer of the present disclosure is polylactide.

In the present disclosure, the biodegradable polymer may be an absorbable polyester copolymer, terpolymer, tetrapolymer, or a mixture thereof. Suitable absorbable polyester copolymers include, but are not limited to, lactide/glycolide copolymer, ε-caprolactone/glycolide copolymer, lactide/trimethylene carbonate copolymer, lactide/glycolide/caprolactone terpolymer, lactide/glycolide/trimethylene carbonate terpolymer, lactide/caprolactone/trimethylene carbonate terpolymer, glycolide/caprolactone/trimethylene carbonate terpolymer, and lactide/glycolide/caprolactone/trimethylene carbonate tetrapolymer.

In some specific embodiments, the biodegradable polymer of the present disclosure is lactide/glycolide copolymer (poly (lactic-co-glycolic acid)). In some more specific embodiments, the molar ratio of lactide to glycolide in the lactide/glycolide copolymer used in the present disclosure is 50:50 to 95:5. Exemplarily, the molar ratio may specifically be 50:50, 75:25, 85:15 or 95:5, etc.

The molecular weight of the biodegradable polymer is not particularly limited in the present disclosure, and may be determined by a person skilled in the art as actually required. In some specific embodiments, the biodegradable polymer of the present disclosure has a molecular weight of 5000 to 70000 Da, preferably 10000 to 50000 Da. Exemplarily, the molecular weight may specifically be 5000 Da, 10000 Da, 15000 Da, 20000 Da, 25000 Da, 30000 Da, 35000 Da, 40000 Da, 45000 Da, 50000 Da, 55000 Da, 60000 Da, 65000 Da, 70000 Da or any numerical value within the range, etc.

In the present disclosure, the content of the biodegradable polymer may vary in a certain range according to specific needs. In some specific embodiments, the content of the biodegradable polymer of the present disclosure is 20% to 50% by mass percentage of the total mass of the gel carrier. Exemplarily, the content of the biodegradable polymer may specifically be 20%, 23%, 25%, 27%, 30%, 31%, 32%, 33%, 34%, 35%, 36%, 37%, 38%, 39%, 40%, 41%, 42%, 43%, 44%, 45%, 46%, 47%, 48%, 49% or 50%, etc. In some more specific embodiments, the content of the biodegradable polymer of the present disclosure is 30% to 50% by mass percentage of the total mass of the gel carrier.

In the present disclosure, the biodegradable polymer may also be a polyetherester, which refers to a polymer in which all or substantially all of the repeating units are linked together by ester groups or ether groups, and in which both ether groups and ester groups are present as linking groups in the polymer. The biodegradable polymer may be a polyether/polyester polymer, which is a polyetherester having a block copolymer structure that includes one or more repeating unit blocks linked together by ether groups and one or more repeating unit blocks linked together by ester groups.

### [Organic Solvent]

In the present disclosure, "organic solvent" refers to a biocompatible organic solvent that may diffuse in an organism and be metabolized or absorbed by the organism, for example, N-methyl pyrrolidone, ethyl acetate, medium-chain triglyceride, glyceryl triacetate, glyceryl tricaprylate, benzyl benzoate, benzyl alcohol, dimethyl sulfoxide, ethyl benzoate, ethanol, glyceraldehyde, and glycerol formal in the art.

In some specific embodiments, the organic solvent of the present disclosure is N-methyl pyrrolidone and/or dimethyl sulfoxide. In some specific embodiments, the organic solvent of the present disclosure is N-methyl pyrrolidone. In other specific embodiments, the organic solvent of the present disclosure is dimethyl sulfoxide. In some specific embodiments, organic solvent of the present disclosure is N-methyl pyrrolidone and dimethyl sulfoxide.

In the present disclosure, the content of the organic solvent may vary in a certain range according to specific needs. In some specific embodiments, the mass ratio of the organic solvent to the hydrophobic additive in the present disclosure is 1:1 to 9:1. Exemplarily, the mass ratio may specifically be 1:1, 2:1, 3:1, 4:1, 5:1, 6:1, 7:1, 7:3, 8:1 or 9:1 etc. In some specific embodiments, the content of the organic solvent of the present disclosure is 20% to 60% by mass of the total mass of the gel carrier. Exemplarily, the content of the organic solvent may specifically be 20%, 25%, 30%, 35%, 39%, 40%, 41%, 42%, 43%, 44%, 45%, 46%, 47%, 48%, 49%, 50%, 51%, 52%, 53%, 54%, 55%, 56%, 57%, 58%, 59% or 60%, etc.

### [Hydrophobic Additive]

In the present disclosure, "hydrophobic additive" may be a pharmaceutically acceptable additive having hydrophobicity and does not include hydrophobic active substances or active ingredients, for example, fatty alcohols such as stearyl alcohol, fatty acids such as sorbic acid, fatty acid esters such as medium-chain triglyceride, and siloxane compounds such as dimethyl siloxane in the art.

In some specific embodiments, the hydrophobic additive of the present disclosure is one or more selected from the group consisting of ethyl acetate, medium-chain triglyceride, glyceryl triacetate, glyceryl tricaprylate, benzyl benzoate, and benzyl alcohol. In some specific embodiments, the hydrophobic additive of the present disclosure is one or more selected from the group consisting of glyceryl triacetate, glyceryl tricaprylate, and benzyl benzoate.

In the present disclosure, the content of the hydrophobic additive may vary in a certain range according to specific needs. In some specific embodiments, the content of the hydrophobic additive of the present disclosure is 1% to 50% by mass percentage of the total mass of the gel carrier. Exemplarily, the content of the hydrophobic additive may specifically be 1%, 5%, 6%, 7%, 8%, 9%, 10%, 11%, 12%, 13%, 14%, 15%, 16%, 17%, 18%, 19%, 20%, 21%, 22%, 23%, 24%, 25%, 30%, 35%, 40%, 45% or 50%, etc. In some more specific embodiments, the content of the hydrophobic additive of the present disclosure is 5% to 30% by mass percentage of the total mass of the gel carrier.

### [Hydrophilic Gel Matrix]

In the present disclosure, "hydrophilic gel matrix" is an excipient used as a carrier material. It is used mainly to regulate the drug release rate and exert a sustained-release or controlled-release effect, so that the rate and amount of drug release in the preparation meet appropriate requirements to ensure that the drug is delivered to the diseased site at a certain rate and maintained at a certain concentration in the body to achieve the desired effect and reduce toxic and side effects.

In some specific embodiments, the gel carrier of the present disclosure comprises a hydrophilic gel matrix. In other specific embodiments, the gel carrier of the present disclosure is devoid of a hydrophilic gel matrix.

In the present disclosure, the hydrophilic gel matrix may be a hydrophilic polymer, e.g., natural gums, such as alginate, agar, xanthan gum, and tragacanth; cellulose derivatives, such as methyl cellulose (MC), sodium carboxymethyl cellulose (CMC-Na), hydroxypropyl methyl cellulose (HPMC), and hydroxyethyl cellulose (HEC); non-cellulosic polysaccharides, such as chitin, chitosan, and carbomer; macromolecular polymers, such as povidone (PVP), ethylene polymers, acrylic resin, and polyvinyl alcohol (PVA).

In some specific embodiments, the hydrophilic gel matrix of the present disclosure is one or more selected from the group consisting of poloxamer, carbomer, polyvinylpyrrolidone, hydroxypropyl methyl cellulose, and sodium carboxymethyl cellulose. In some more specific embodiments, the hydrophilic gel matrix of the present disclosure is one or more selected from the group consisting of poloxamer 188, carbomer, and polyvinylpyrrolidone.

In the present disclosure, the content of the hydrophilic gel matrix may vary in a certain range according to specific needs. In some specific embodiments, the content of the hydrophilic gel matrix of the present disclosure is 0.5% to 15% by mass percentage of the total mass of the gel carrier. Exemplarily, the content of the hydrophilic gel matrix may specifically be 0.5%, 1%, 2%, 3%, 4%, 5%, 7%, 10%, 12% or 15%, etc. In some more specific embodiments, the content of the hydrophilic gel matrix of the present disclosure is 1% to 5% by mass percentage of the total mass of the gel carrier.

### <Second Aspect>

In the second aspect, the present disclosure provides a delivery preparation, comprising an active pharmaceutical ingredient and the gel carrier provided in the <First Aspect> described above of the present disclosure.

The terms used for or involved in this aspect, if disclosed in the <First Aspect>, have the same meanings as disclosed or defined in the previous aspect.

### [Delivery Preparation]

In the present disclosure, "delivery preparation" is a pharmaceutical composition, comprising an active pharmaceutical ingredient (API) and a gel carrier (in some cases, it comprises only an active pharmaceutical ingredient and a gel carrier), which may release the active pharmaceutical ingredient in a particular release behavior (e.g., stably, slowly, and continuously) after being administered to a subject.

In some specific embodiments, the delivery preparation of the present disclosure comprises an active pharmaceutical ingredient and a gel carrier, wherein the gel carrier comprises a biodegradable polymer, an organic solvent, a hydrophobic additive, and optionally a hydrophilic gel matrix. In some more specific embodiments, the delivery preparation of the present disclosure comprises an active pharmaceutical ingredient, a biodegradable polymer, an organic solvent, and a hydrophobic additive. In other more specific embodiments, the delivery preparation of the present disclosure comprises an active pharmaceutical ingredient, a biodegradable polymer, an organic solvent, a hydrophobic additive, and a hydrophilic gel matrix.

### [Active Pharmaceutical Ingredient]

In the present disclosure, "active pharmaceutical ingredient" refers to any one or a mixture of the substances in the delivery preparation, and such substances have pharmacological or other direct effects on the diagnosis, treatment, symptom relief or management of disease or the prevention of disease or can affect the function or structure of an organism.

In some embodiments, the active pharmaceutical ingredient of the present disclosure may be a drug for the treatment of schizophrenia or a hormone drug. In some specific embodiments, the active pharmaceutical ingredient of the present disclosure includes, but is not limited to, risperidone, paliperidone, bupivacaine, ropivacaine, leuprorelin, triptorelin, ktreotide, rotigotine, pramipexole, lumateperone or a pharmaceutically acceptable salt thereof. In some specific embodiments, the active pharmaceutical ingredient of the present disclosure is rotigotine, pramipexole, lumateperone or a pharmaceutically acceptable salt thereof, etc.

In the present disclosure, the content of the active pharmaceutical ingredient may vary in a certain range according to specific needs. In some specific embodiments, the content of the active pharmaceutical ingredient is always calculated in its free base form. In other specific embodiments, the content of the active pharmaceutical ingredient is always calculated in the form of its acid addition salt. In some specific embodiments, the content of the active pharmaceutical ingredient (e.g., calculated in its free base form) in the delivery preparation of the present disclosure is 0.5% to 30% by mass percentage. Exemplarily, the content of the active pharmaceutical ingredient may specifically be 0.5%, 1%, 2%, 3%, 3.1%, 3.2%, 3.3%, 3.4%, 3.5%, 3.6%, 3.7%, 3.8%, 3.9%, 4%, 4.1%, 4.2%, 4.3%, 4.4%, 4.5%, 5%, 5.1%, 5.2%, 5.3%, 5.4%, 5.5%, 6%, 6.1%, 6.2%, 6.3%, 6.4%, 6.5%, 6.6%, 6.7%, 6.8%, 6.9%, 7%, 7.1%, 7.2%, 7.3%, 7.4%, 7.5%, 8%, 8.5%, 9%, 9.1%, 9.2%, 9.3%, 9.4%, 9.5%, 10%, 11%, 12%, 13%, 14%, 15%, 16%, 17%, 18%, 19%, 20%, 21%, 22%, 23%, 24%, 25%, 26%, 27%, 28%, 29% or 30%, etc. In some more specific embodiments, the content of the active pharmaceutical ingredient (e.g., calculated in its free base form) in the delivery preparation of the present disclosure is 3% to 20% by mass percentage.

### <Third Aspect>

In the third aspect, the present disclosure provides a preparation method for the gel carrier provided in the <First Aspect> described above of the present disclosure.

The terms used for or involved in this aspect, if disclosed in the <First Aspect>, have the same meanings as disclosed or defined in the previous aspect.

In some specific embodiments, the preparation method for the gel carrier of the present disclosure may comprise the following steps: weighing prescribed doses of a biodegradable polymer, an organic solvent, a hydrophobic additive, and optionally a hydrophilic gel matrix, and mixing to homogeneity to obtain the gel carrier.

### <Fourth Aspect>

In the fourth aspect, the present disclosure provides a preparation method for the delivery preparation provided in the <Second Aspect> described above of the present disclosure.

The terms used for or involved in this aspect, if disclosed in the <First Aspect> or <Second Aspect>, have the same meanings as disclosed or defined in the previous aspects.

In some specific embodiments, the preparation method for the delivery preparation of the present disclosure may comprise the following steps: weighing prescribed doses of a biodegradable polymer, an organic solvent, a hydrophobic additive, and optionally a hydrophilic gel matrix, and mixing to homogeneity, and then adding an active pharmaceutical ingredient (e.g., a prescribed dose of the active pharmaceutical ingredient), and mixing to homogeneity to obtain the delivery preparation.

In some preferred embodiments, the preparation method for the delivery preparation of the present disclosure may comprise the following steps: weighing prescribed doses of a biodegradable polymer, an organic solvent, a hydrophobic additive, and optionally a hydrophilic gel matrix, and mixing to homogeneity, and adding an active pharmaceutical ingredient (e.g., a prescribed dose of the active pharmaceutical ingredient) just before administration, and mixing to homogeneity to obtain the delivery preparation. In this embodiment, the active pharmaceutical ingredient is added just before administration, which contributes to a guarantee of the stability of the delivery preparation.

In other preferred embodiments, the preparation method for the delivery preparation of the present disclosure may comprise the following steps: weighing prescribed doses of a biodegradable polymer, an organic solvent, a hydrophobic additive, and optionally a hydrophilic gel matrix, and mixing to homogeneity, and immediately adding an active pharmaceutical ingredient (e.g., a prescribed dose of the active pharmaceutical ingredient), and mixing to homogeneity to obtain the delivery preparation. In this embodiment, there is no need to add the active pharmaceutical ingredient just before administration, and the resulting delivery preparation containing the active pharmaceutical ingredient can be stored stably, while improving the convenience in use of the delivery preparation.

In other specific embodiments, the preparation method for the delivery preparation of the present disclosure may comprise the following steps: weighing prescribed doses of a biodegradable polymer, an active pharmaceutical ingredient, an organic solvent, a hydrophobic additive, and optionally a hydrophilic gel matrix, and mixing to homogeneity to obtain the delivery preparation. In this embodiment, there is no need to dissolve and mix the ingredients of the gel carrier in advance because they can be directly mixed with the active pharmaceutical ingredient to homogeneity. As a result, the order of dissolving various ingredients and the timing of adding the active pharmaceutical ingredient are not necessarily considered, and meanwhile, the stability of delivery preparations during storage will not be negatively affected, which greatly improves the convenience in storage, transportation, and use of the delivery preparation.

The composition of the delivery preparation of the present disclosure is as shown in FIG. 3. To express the technical solution of the present disclosure more clearly, it will be further described below with reference to specific examples, which cannot be used to limit the scope of the present disclosure but serve only as part of examples of the present disclosure. Unless otherwise stated, all of the instruments, reagents, materials, experimental animals, etc. used for or involved in the present disclosure are commercially available through conventional means.

### Example 1

0.490 g of PLGA (7525 DLG 2A) and 0.210 g of PLGA (5050 DLG 4.5A) were precisely weighed, and 1.060 g of NMP was added, and dissolved while stirring to obtain a clear and transparent gel solution. Just before administration, 0.120 g of rotigotine was added to the solution prepared above, and mixed to homogeneity to obtain the product.

### Example 2

0.490 g of PLGA (7525 DLG 2A) and 0.210 g of PLGA (5050 DLG 4.5A) were precisely weighed, and 0.954 g of NMP and 0.106 g of benzyl benzoate were added, and dissolved while stirring to obtain a clear and transparent gel solution. Just before administration, 0.120 of rotigotine was added to the solution prepared above and mixed to homogeneity to obtain the product.

### Example 3

0.490 g of PLGA (7525 DLG 2A) and 0.210 g of PLGA (5050 DLG 4.5A) were precisely weighed, and 0.848 g of NMP and 0.212 g of benzyl benzoate were added, and dissolved while stirring to obtain a clear and transparent gel solution. Just before administration, 0.120 g of rotigotine was added to the solution prepared above and mixed to homogeneity to obtain the product.

### Example 4

0.490 g of PLGA (7525 DLG 2A) and 0.210 g of PLGA (5050 DLG 4.5A) were precisely weighed, and 0.848 g of NMP and 0.212 g of glyceryl tricaprylate were added, and dissolved while stirring to obtain a clear and transparent gel solution. Just before administration, 0.120 g of rotigotine was added to the solution prepared above and mixed to homogeneity to obtain the product.

### Example 5

0.490 g of PLGA (7525 DLG 2A) and 0.210 g of PLGA (5050 DLG 4.5A) were precisely weighed, and 0.848 g of NMP and 0.212 g of glyceryl triacetate were added, and dissolved while stirring to obtain a clear and transparent gel solution. Just before administration, 0.120 g of rotigotine was added to the solution prepared above and mixed to homogeneity to obtain the product.

### Example 6

0.572 g of PLGA (5050 DLG 4.5A) was precisely weighed, and 0.954 g of NMP and 0.106 g of benzyl benzoate were added, and dissolved while stirring to obtain a clear and transparent gel solution. Just before administration, 0.120 g of rotigotine was added to the solution prepared above and mixed to homogeneity to obtain the product.

### Example 7

0.572 g of PLGA (5050 DLG 4.5A) and 0.088 g of poloxamer 188 were precisely weighed, and 0.954 g of NMP and 0.106 g of benzyl benzoate were added, and dissolved while stirring to obtain a clear and transparent gel solution. Just before administration, 0.120 g of rotigotine was added to the solution prepared above and mixed to homogeneity to obtain the product.

### Example 8

0.572 g of PLGA (5050 DLG 4.5A) and 0.088 g of polyvinylpyrrolidone were precisely weighed, and 0.954 g of NMP and 0.106 g of benzyl benzoate were added, and dissolved while stirring to obtain a clear and transparent gel solution. Just before administration, 0.120 g of rotigotine was added to the solution prepared above and mixed to homogeneity to obtain the product.

### Example 9

0.572 g of PLGA (5050 DLG 4.5A) and 0.018 g of carbomer were precisely weighed, and 0.954 g of NMP and 0.106 g of benzyl benzoate were added, and dissolved while stirring to obtain a clear and transparent gel solution. Just before administration, 0.120 g of rotigotine was added to the solution prepared above and mixed to homogeneity to obtain the product.

### Example 10

0.525 g of PLGA (5050 DLG 2E) was precisely weighed, and 0.555 g of NMP and 0.240 g of benzyl benzoate were added, and dissolved while stirring to obtain a clear and transparent solution. Just before administration, 0.090 g of rotigotine was added to the solution prepared above and mixed to homogeneity to obtain the product.

### Example 11

0.345 g of PLGA (5050 DLG 4.5A) was precisely weighed, and 0.555 g of NMP and 0.240 g of benzyl benzoate were added, and dissolved while stirring to obtain a clear and transparent solution. Just before administration, 0.090 g of rotigotine was added to the solution prepared above and mixed to homogeneity to obtain the product.

### Example 12

0.345 g of PLGA (7525 DLG 5.5E) was precisely weighed, and 0.555 g of NMP and 0.240 g of benzyl benzoate were added, and dissolved while stirring to obtain a clear and transparent solution. Just before administration, 0.090 g of rotigotine was added to the solution prepared above and mixed to homogeneity to obtain the product.

### Example 13

0.525 g of PLGA (5050 DLG 4.5A) was precisely weighed, and 0.683 g of NMP and 0.293 g of benzyl benzoate were added, and dissolved while stirring to obtain a clear and transparent solution. Just before administration, 0.055 g of rotigotine was added to the solution prepared above and mixed to homogeneity to obtain the product.

### Example 14

0.525 g of PLGA (7525 DLG 5.5E) was precisely weighed, and 0.683 g of NMP and 0.293 g of benzyl benzoate were added, and dissolved while stirring to obtain a clear and transparent solution. Just before administration, 0.055 g of rotigotine was added to the solution prepared above and mixed to homogeneity to obtain the product.

### Example 15

0.675 g of PLGA (7525 DLG 2A) was precisely weighed, and 0.578 g of NMP and 0.248 g of benzyl benzoate were added, and dissolved while stirring to obtain a clear and transparent solution. Just before administration, 0.055 g of rotigotine was added to the solution prepared above and mixed to homogeneity to obtain the product.

### Example 16

0.675 g of PLGA (100 DL 2A) was precisely weighed, and 0.578 g of NMP and 0.248 g of benzyl benzoate were added, and dissolved while stirring to obtain a clear and transparent solution. Just before administration, 0.055 g of rotigotine was added to the solution prepared above and mixed to homogeneity to obtain the product.

### Example 17

0.245 g of PLGA (7525 DLG 2A) and 0.105 g of PLGA (5050 DLG 4.5A) were precisely weighed, and 0.477 g of NMP and 0.053 g of benzyl benzoate were added, and dissolved while stirring to obtain a clear and transparent solution. Just before administration, 0.090 g of pramipexole was added to the solution prepared above and mixed to homogeneity to obtain the product.

### Example 18

0.245 g of PLGA (7525 DLG 2A) and 0.105 g of PLGA (5050 DLG 4.5A) were precisely weighed, and 0.477 g of NMP and 0.053 g of glyceryl triacetate were added, and dissolved while stirring to obtain a clear and transparent solution. Just before administration, 0.090 g of pramipexole was added to the solution prepared above and mixed to homogeneity to obtain the product.

### Example 19

0.540 g of PLGA (5050 DLG 2E) was precisely weighed, and 0.810 g of NMP was added, and dissolved while stirring to obtain a clear and transparent solution. Just before administration, 0.082 g of pramipexole pamoate (API:acid=2:1) was added to the solution prepared above and mixed to homogeneity to obtain the product.

### Example 20

0.572 g of PLGA (5050 DLG 2E) and 0.088 g of poloxamer188 were precisely weighed, and 0.954 g of NMP and 0.106 g of benzyl benzoate were added, and dissolved while stirring to obtain a clear and transparent solution. Just before administration, 0.108 g of pramipexole pamoate (API:acid=2:1) was added to the solution prepared above and mixed to homogeneity to obtain the product.

### Example 21

0.540 g of PLGA (5050 DLG 2E) was precisely weighed, and 0.810 g of NMP was added, and dissolved while stirring to obtain a clear and transparent solution. Just before administration, 0.120 g of pramipexole pamoate (API:acid=1:1) was added to the solution prepared above and mixed to homogeneity to obtain the product.

### Example 22

0.572 g of PLGA (5050 DLG 2E) and 0. 088 g of polyvinylpyrrolidone were precisely weighed, and 0.954 g of NMP and 0.106 g of benzyl benzoate were added, and dissolved while stirring to obtain a clear and transparent solution. Just before administration, 0.156 g of pramipexole pamoate (API:acid=1:1) was added to the solution prepared above and mixed to homogeneity to obtain the product.

### Example 23

0.540 g of PLGA (5050 DLG 2E) was precisely weighed, and 0.810 g of NMP was added, and dissolved while stirring to obtain a clear and transparent solution. Just before administration, 0.096 g of pramipexole palmitate was added to the solution prepared above and mixed to homogeneity to obtain the product.

### Example 24

0.572 g of PLGA (5050 DLG 2E) and 0.018 g of carbomer were precisely weighed, and 0.954 g of NMP and 0.106 g of benzyl benzoate were added, and dissolved while stirring to obtain a clear and transparent solution. Just before administration, 0.118 g of pramipexole palmitate was added to the solution prepared above and mixed to homogeneity to obtain the product.

### Example 25

0.350 g of PLGA (5050 DLG 4.5A) was precisely weighed, and 0.477 g of NMP was added and stirred until they were dissolved to obtain a clear and transparent solution. Just before administration, 0.220 g of lumateperone was added to the solution prepared above and mixed to homogeneity to obtain the product.

### Example 26

0.350 g of PLGA (5050 DLG 4.5A) was precisely weighed, and 0.477 g of NMP and 0.053 g of glyceryl triacetate were added and stirred until they were dissolved to obtain a clear and transparent solution. Just before administration, 0.220 g of lumateperone was added to the solution prepared above and mixed to homogeneity to obtain the product.

### Example 27

0.213 g of PLGA (5050 DLG 4.5A) and 0.491 g of PLGA (7525 DLG 2A) were precisely weighed, and 0.965 g of NMP and 0.104 g of glyceryl triacetate were added and stirred until they were dissolved to obtain a clear and transparent solution. Just before administration, 0.438 g of lumateperone was added to the solution prepared above and mixed to homogeneity to obtain the product.

### Example 28

0.210 g of PLGA (5050 DLG 4.5A) and 0.490 g of PLGA (7525 DLG 2A) were precisely weighed, and 0.954 g of NMP and 0.106 g of glyceryl triacetate were added and stirred until they were dissolved to obtain a clear and transparent solution. Just before administration, 0.142 g of lumateperone was added to the solution prepared above and mixed to homogeneity to obtain the product.

### Example 29

0.675 g of PLGA (7525 DLG 2A) was precisely weighed, and 0.826 g of NMP was added, and dissolved while stirring to obtain a clear and transparent solution. Just before administration, 0.055 g of rotigotine was added to the solution prepared above and mixed to homogeneity to obtain the product.

### Example 30

0.675g PLGA (8515 DLG 2A) was precisely weighed, and 0.578 g of NMP and 0.248 g of benzyl benzoate were added, and dissolved while stirring to obtain a clear and transparent solution. Just before administration, 0.055 g of rotigotine was added to the solution prepared above and mixed to homogeneity to obtain the product.

### Example 31

0.675g PLGA (7525 DLG 2A) was precisely weighed, 0.578 g of NMP and 0.248 g of benzyl benzoate were added, and dissolved while stirring to obtain a clear and transparent solution, and then 0.055 g of rotigotine was added and mixed to homogeneity to obtain the product.

### Example 32

0.675 g of PLGA (7525 DLG 2A), 0.578 g of NMP, 0.248 g of benzyl benzoate, and 0.055 g of rotigotine were precisely weighed, and mixed and dissolved by stirring to homogeneity to obtain the product.

The composition of formulations of the above Examples were listed in Table 1 below.

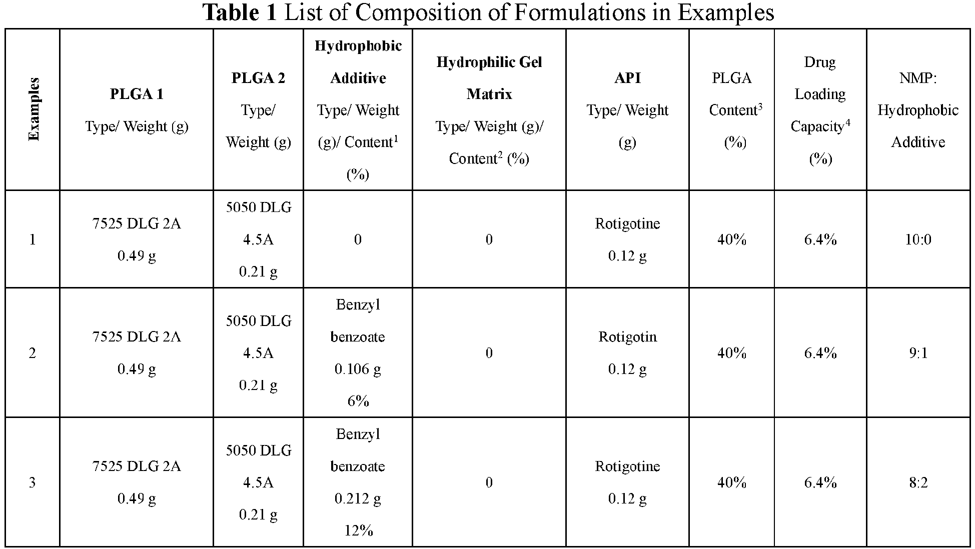

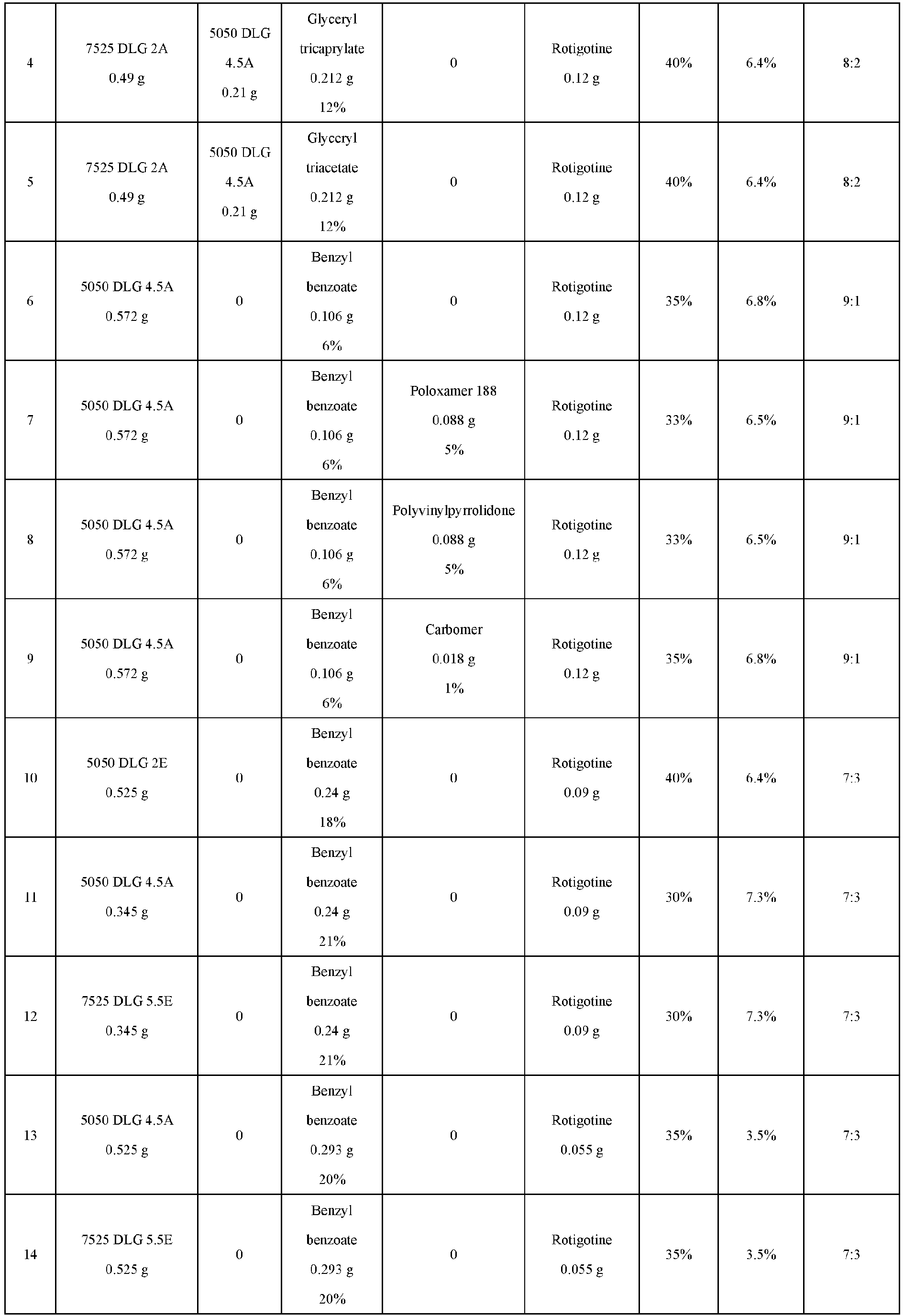

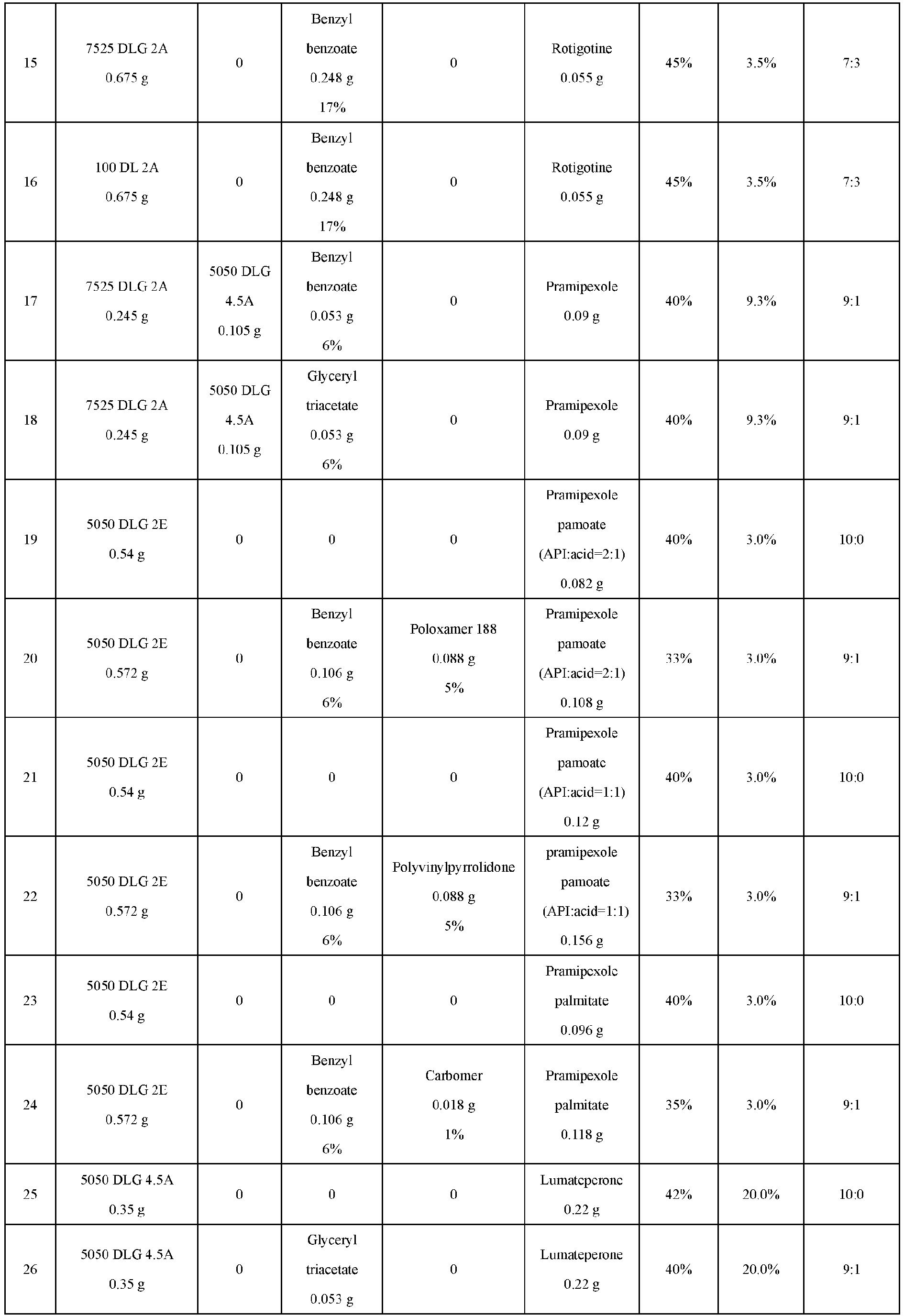

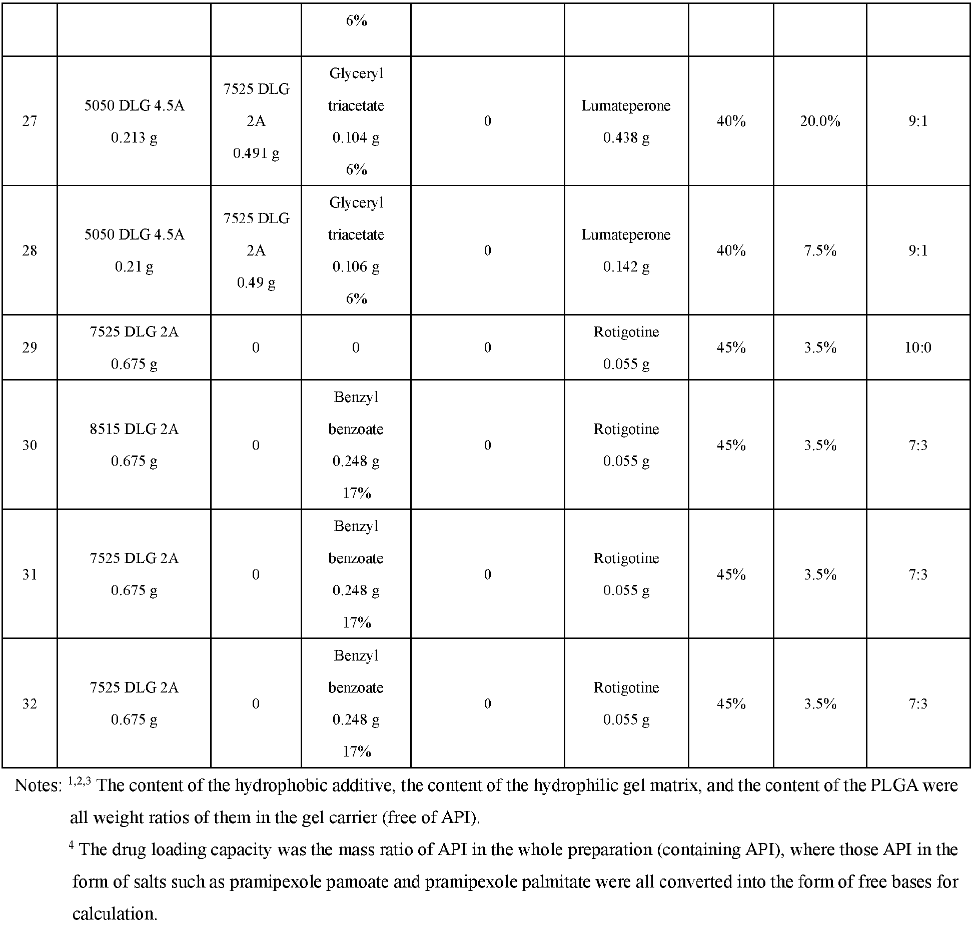

### Experimental Example 1: Study on viscosity of in situ gel preparations

Approach 3 in General Principles 0633 of *Chinese Pharmacopoeia (Volume IV)* was followed. A cone digital viscometer was used for determination. 500 µL of the sample solution was precisely measured and tested at 25°C using a 40# rotor or 52# rotor. The revolutions were adjusted such that the torque was in the range of 25% to 75%. Each sample was tested twice in parallel. The measured viscosity values were as shown in the table below.

**Table 2 Test Results of Viscosities of Rotigotine-In Situ Gel**

| | PLGA Concentration (%) | PLGA Type | Temp. (°C) | Viscosity (CP) |
|---|---|---|---|---|
| Example 10 | 40 | 5050 DLG 2E | 25 | 1851 |
| Example 11 | 30 | 5050 DLG 4.5A | 25 | 6349 |
| Example 12 | 30 | 7525 DLG 5.5E | 25 | 7756 |
| Example 13 | 35 | 5050 DLG 4.5A | 25 | 17865 |
| Example 14 | 35 | 7525 DLG 5.5E | 25 | 19409 |
| Example 15 | 45 | 7525 DLG 2A | 25 | 1988 |
| Example 29 | 45 | 7525 DLG 2A | 25 | 1156 |

The results of Examples 10 to 15 and 29 showed that the viscosity of the preparation was related mainly to the PLGA molecular weight and concentration, namely, the higher the PLGA molecular weight or concentration, the greater the viscosity of the preparation. Furthermore, the hydrophobic additive also increased the viscosity of the preparation. As could be appreciated from the release results, under the same circumstances, the greater the viscosity of the preparation, the slower the drug release *in vitro,* which might be mainly because the increase in viscosity rendered the gel carrier denser, increasing the resistance of the carrier to the drug, so that it was difficult for external media to enter the gel matrix and the degradation of PLGA slowed down, thereby retarding the release. When the preparations had the same or similar drug loading capacities, both the gel viscosity and the ratio of lactide:glycolide in the polymer would affect the drug release effect.

### Experimental Example 2: Study on in vitro release characteristics of in situ gel preparations

### (1) Study on in vitro release characteristics of rotigotine-in situ gel preparations

A 1-mL syringe connected with a 21G needle (outer diameter: 0.8 mm) was utilized to aspirate 0.1 mL of samples, and precisely weighed. The samples were slowly injected into a release medium (0.01M phosphate buffer solution, containing 0.02% NaN₃ and 0.2% SDS, adjusting the pH to 7.40). The volume of the release medium was adjusted according to the sink condition. The syringe was precisely weighed again. The injected amount of the samples was calculated by the decrement method. The sample vials were placed in a constant temperature oscillating water bath at 37 ± 0.5°C and oscillated at 50 rpm. Triplicates were prepared for each sample. 2 mL of supernatant was collected at the predetermined points in time, while supplementing 2 mL of the release media at the same temperature. The above test solution was collected and tested by HPLC. The cumulative amount of release was calculated according to the external standard method.

### (2) Study on in vitro release characteristics of pramipexole-in situ gel preparations

A 1-mL syringe connected with a 21G needle (outer diameter: 0.8 mm) was utilized to aspirate 0.1 mL of samples, and precisely weighed. The samples were slowly injected into a release medium (0.01M phosphate buffer solution, containing 0.02% NaN₃ and 0.2% SDS, adjusting the pH to 7.40). The volume of the release medium was adjusted according to the sink condition. The syringe was precisely weighed again. The injected amount of the samples was calculated by the decrement method. The sample vials were placed in a constant temperature oscillating water bath at 37 ± 0.5°C and oscillated at 50 rpm. Triplicates were prepared for each sample. 2 mL of supernatant was collected at the predetermined points in time, while supplementing 2 mL of the release media at the same temperature. The above test solution was collected and tested by HPLC. The cumulative amount of release was calculated according to the external standard method.

### (3) Study on in vitro release characteristics of lumateperone-in situ gel preparations

A 1-mL syringe connected with a 21G needle (outer diameter: 0.8 mm) was utilized to aspirate 0.04 mL of samples, and precisely weighed. The samples were slowly injected into a release medium (0.01M phosphate buffer solution, containing 0.02% NaN₃ and 0.2% SDS, adjusting the pH to 7.40). The volume of the release medium was adjusted according to the sink condition. The syringe was precisely weighed again. The injected amount of the samples was calculated by the decrement method. The sample vials were placed in a constant temperature oscillating water bath at 37 ± 0.5°C and oscillated at 50 rpm. Triplicates were prepared for each sample. 2 mL of supernatant was collected at the predetermined points in time, while supplementing 2 mL of the release media at the same temperature. The above test solution was collected and tested by HPLC. The cumulative amount of release was calculated according to the external standard method.

**Table 3 Experimental Results of In Vitro Release Characteristics of Preparations in Some Examples**

| Examples | API Types | *In Vitro* Release Characteristics % | | | | | |
|---|---|---|---|---|---|---|---|
| | | 1h | 24h | 7d | 14d | 21d | 28d |
| Example 1 | Rotigotine | 1.4 | 10.4 | 36.7 | 57.2 | / | / |
| Example 2 | Rotigotine | 0.9 | 7.1 | 26.5 | 49.7 | / | / |
| Example 3 | Rotigotine | 0.7 | 10.9 | 22.5 | 39.1 | / | / |
| Example 4 | Rotigotine | 0.5 | 6.9 | 23.6 | 43.7 | / | / |
| Example 5 | Rotigotine | 1.2 | 8.8 | 26.2 | 54.3 | 60.5 | 68.5 |
| Example 6 | Rotigotine | 1.0 | 6.3 | 16.1 | 68.5 | 83.4 | 96.3 |
| Example 7 | Rotigotine | 0.7 | 6.2 | 15.9 | 57.5 | 74.9 | 89.0 |
| Example 8 | Rotigotine | 0.8 | 9.3 | 18.6 | 50.2 | 76.3 | 83.0 |
| Example 9 | Rotigotine | 0.6 | 8.7 | 20.5 | 47.5 | 70.2 | 95.2 |
| Example 10 | Rotigotine | 0.8 | 11.5 | 39.2 | 61.7 | 81.2 | 90.1 |
| Example 11 | Rotigotine | 1.0 | 6.5 | 27.0 | 47.1 | 70.7 | 85.6 |
| Example 12 | Rotigotine | 0.8 | 9.1 | 16.9 | 32.2 | 45.7 | 60.2 |
| Example 13 | Rotigotine | 2.4 | 13.7 | / | / | / | / |
| Example 14 | Rotigotine | 1.6 | 9.6 | / | / | / | / |
| Example 15 | Rotigotine | 1.7 | 10.0 | 29.4 | 55.5 | 72.5 | 81.6 |
| Example 16 | Rotigotine | 2.4 | 13.7 | 31.7 | 44.8 | 62.2 | 65.5 |
| Example 19 | Pramipexole pamoate (API:acid=2:1) | 11.8 | 36.7 | 76.6 | 82.1 | / | / |
| Example 20 | Pramipexole pamoate (API:acid=2:1) | 3.4 | 16.6 | 38.6 | 56.4 | 65.3 | 72.6 |
| Example 21 | Pramipexole pamoate (API:acid=1:1) | 28.5 | 34.8 | 86.7 | / | / | / |
| Example 22 | Pramipexole pamoate (API:acid=1:1) | 5.7 | 24.9 | 73.4 | 86.5 | 97.4 | / |
| Example 23 | Pramipexole palmitate | 7.6 | 27.2 | 97.8 | / | / | / |
| Example 24 | Pramipexole palmitate | 4.4 | 25.5 | 72.9 | 84.9 | 96.7 | / |
| Example 25 | Lumateperone | 8.1 | 48.6 | 74.9 | 76.8 | / | / |
| Example 26 | Lumateperone | 2.4 | 34.2 | 69.5 | 75.6 | 76.3 | / |
| Example 27 | Lumateperone | 2.1 | 25.4 | 52.3 | 72.9 | 79.7 | / |
| Example 28 | Lumateperone | 1.6 | 22.3 | 46.5 | 68.6 | 75.3 | / |
| Example 29 | Rotigotine | 2.4 | 14.7 | / | / | / | / |
| Example 30 | Rotigotine | 1.6 | 11.1 | / | / | / | / |

Among Examples 1 to 5, the 1 h/24 h burst releases of the preparations in Examples 2 to 5 were significantly reduced after the addition of a certain proportion of a hydrophobic additive such as benzyl benzoate, glyceryl triacetate or glyceryl tricaprylate, as compared to Example 1. Among Examples 6 to 9, the 1 h burst releases in Examples 7 to 9 were remarkably reduced after the addition of a small amount of a hydrophilic gel matrix, as compared to Example 6. In Examples 10 to 12, different types of PLGAs were screened to study their influences on the release characteristics of the preparations. The 24 h burst release of the preparation in the 5050 DLG 4.5A group was smaller. The release period was related mainly to the molecular weight of the polymer, namely, the release period was significantly prolonged with the increase in the molecular weight. The release periods of the preparations in the 5050 DLG 4.5A group and the 7525 DLG 5.5E group were significantly longer than that in the 5050 2E group, with the 7525 DLG 5.5E group having the longest release period. In Examples 13 to 16, 29, and 30, the PLGA concentration was increased while reducing the drug loading capacity as compared to Examples 10 to 12, so as to further screen the optimal formulation combinations with different PLGAs, and the results showed that the 1 h/24 h *in vitro* burst releases of the preparations were increased as compared to Examples 10 to 12. The hydrophobic additive could significantly reduce the burst release, but different types of PLGAs exerted different influences on the burst release, and both the molecular weight of the polymer and the ratio of lactide to glycolide would affect the burst release effect. Among Examples 19 to 24, the 1 h/24 h burst releases of the drugs in Examples 20, 22, and 24 were significantly reduced after the addition of certain proportions of the hydrophobic additive and hydrophilic gel matrix as compared to the corresponding Examples 19, 21, and 23, and the release periods were prolonged and the *in vivo* plasma concentration could be maintained in a safe and effective range for more than one week. Among Examples 25 to 28, the 1 h/24 h burst releases in Examples 26 to 28 were reduced after the addition of a certain proportion of the hydrophobic additive as compared to Example 25.

### Experimental Example 3: Study on in vivo release characteristics of in situ gel preparations in rats

### (1) Study on in vivo release characteristics of rotigotine-in situ gel preparations in rats

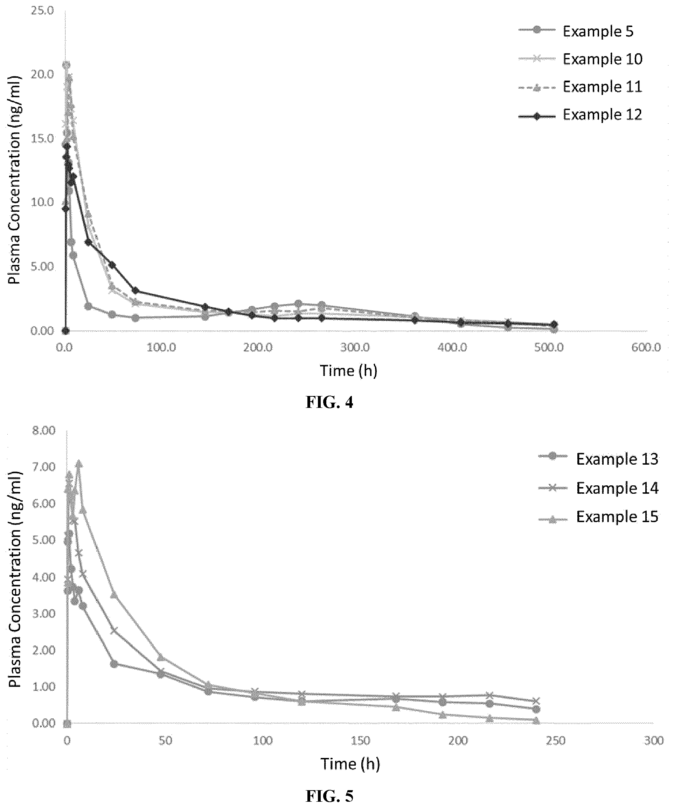

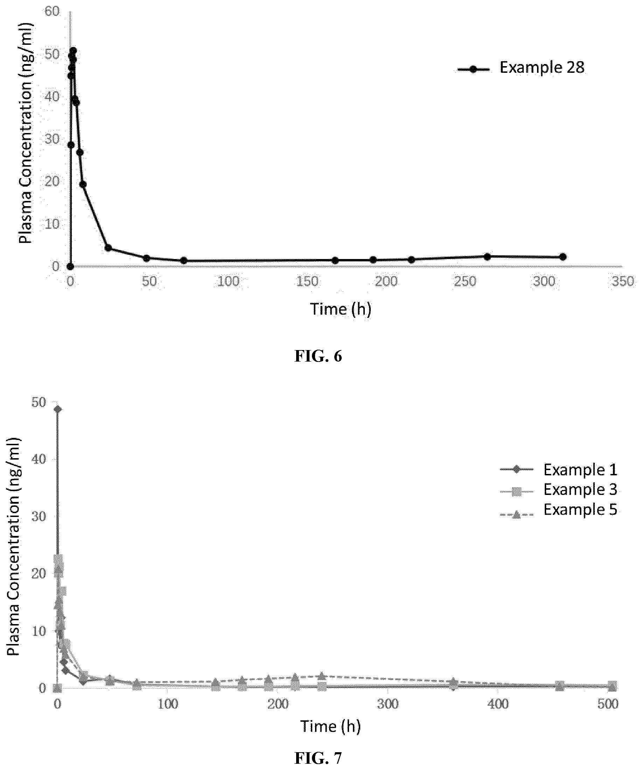

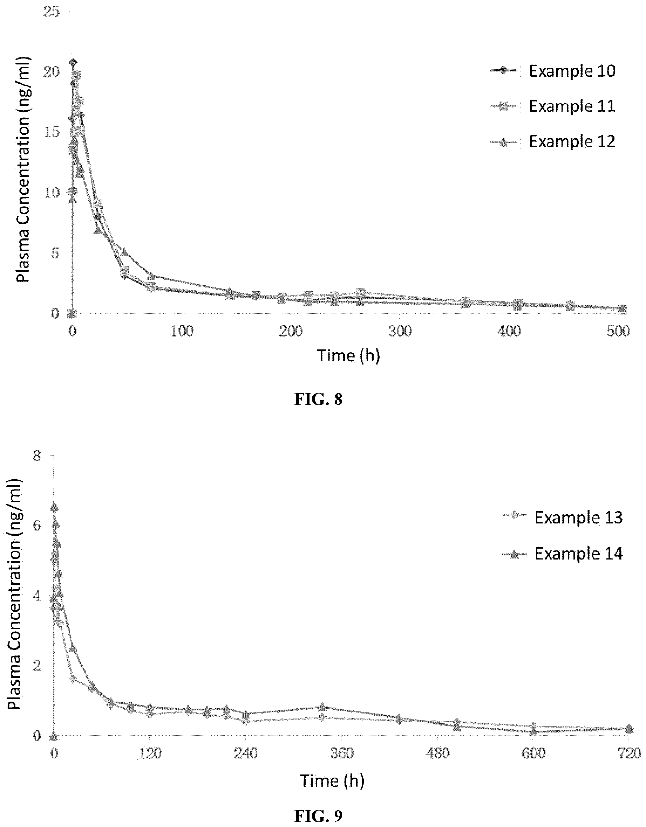

SD male rats (N=5) were selected to study the nonlinear pharmacokinetic (PK) behavior *in vivo.* The dosage was 10 mg/kg (calculated as rotigotine). The preparations of some of the above Examples were injected subcutaneously. Blood was sampled from the tail vein at predetermined points in time. After the plasma samples were treated by Solid-Phase Extraction (SPE), the rotigotine concentration was determined by the LC-MS/MS method. The curves of plasma concentration vs. time (within 504 h) for the samples of Examples 5 and 10 to 12 in rats were shown in FIG. 4. The curves of plasma concentration vs. time (within 504 h) for the samples of Examples 1, 3, and 5 were shown in FIG. 7. The curves of plasma concentration vs. time (within 504 h) for the samples of Examples 10 to 12 were shown in FIG. 8.

**Table 4 Plasma Concentrations of Rotiotine-In Situ Gels in Rats (Examples 1 3 5, 10-12**

| Time (h) | Plasma Concentrations (ng/mL) | | | | | |
|---|---|---|---|---|---|---|
| | Example 1 | Example 3 | Example 5 | Example 10 | Example 11 | Example 12 |
| 0.000 | 0.00 | 0.00 | 0.00 | 0.00 | 0.00 | 0.00 |
| 0.5 | 48.8 | 22.6 | 14.5 | 16.14 | 10.11 | 9.50 |
| 1.0 | 20.9 | 20.2 | 20.7 | 20.79 | 13.68 | 13.53 |
| 2.0 | 9.94 | 21.2 | 15.4 | 19.04 | 15.02 | 14.38 |
| 3.0 | 7.50 | 11.0 | 13.1 | 19.56 | 17.03 | 12.96 |
| 4.0 | 12.3 | 17.0 | 10.9 | 19.82 | 19.75 | 12.66 |
| 6.0 | 4.57 | 7.85 | 6.92 | 17.32 | 17.65 | 11.57 |
| 8.0 | 3.12 | 7.65 | 5.89 | 16.40 | 15.19 | 12.06 |
| 24 | 1.22 | 2.29 | 1.93 | 8.07 | 9.10 | 6.93 |
| 48 | 1.61 | 1.29 | 1.23 | 3.16 | 3.53 | 5.14 |
| 72 | 0.58 | 0.46 | 1.00 | 2.09 | 2.26 | 3.14 |
| 144 | 0.24 | 0.29 | 1.12 | 1.46 | 1.55 | 1.87 |
| 168 | 0.21 | 0.29 | 1.40 | 1.43 | 1.53 | 1.48 |
| 192 | 0.21 | 0.30 | 1.64 | 1.26 | 1.42 | 1.19 |
| 216 | 0.25 | 0.41 | 1.90 | 1.13 | 1.55 | 0.96 |
| 240 | 0.21 | 0.36 | 2.10 | 1.33 | 1.52 | 0.98 |
| 264 | / | / | 2.00 | 1.35 | 1.78 | 0.97 |
| 360 | 0.27 | 0.65 | 1.13 | 1.06 | 1.01 | 0.81 |
| 408 | / | / | 0.55 | 0.84 | 0.80 | 0.64 |
| 456 | 0.32 | 0.56 | 0.27 | 0.70 | 0.65 | 0.56 |
| 504 | 0.31 | 0.50 | 0.13 | 0.45 | 0.35 | 0.48 |

### (2) Study on in vivo release characteristics of rotigotine-in situ gel preparations in rats

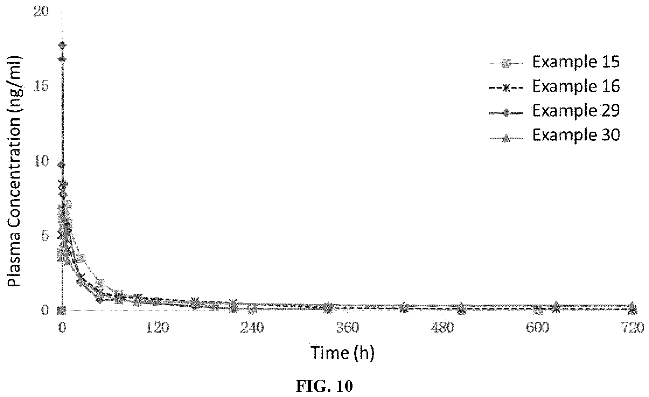

SD male rats (N=5) were selected to study the PK behavior *in vivo.* The dosage was 5 mg/kg (calculated as rotigotine). The preparations of some of the above Examples were injected subcutaneously. Blood was sampled from the tail vein at predetermined points in time. After the plasma samples were treated by Solid-Phase Extraction (SPE), the rotigotine concentration was determined by the LC-MS/MS method. The curves of plasma concentration vs. time (within 720 h) for the samples of Examples 13 and 14 in rats were shown in FIG. 9. The curves of plasma concentration vs. time (within 720 h) for the samples of Examples 15, 16, 29, and 30 were shown in FIG. 10. The curves of plasma concentration vs. time (within 240 h) for the samples of Examples 13 to 15 were shown in FIG. 5.

**Table 5 Plasma Concentrations of Rotiotine-In Situ Gels in Rats (Examples 13-16, 29-30)**

| Time (h) | Plasma Concentrations (ng/mL) | | | | | |
|---|---|---|---|---|---|---|
| | Example 13 | Example 14 | Example 15 | Example 16 | Example 29 | Example 30 |
| 0 | 0.00 | 0.00 | 0.00 | 0.00 | 0.00 | 0.00 |
| 0.25 | 3.63 | 3.95 | 3.85 | 5.05 | 9.76 | 3.57 |
| 0.5 | 4.96 | 5.13 | 6.41 | 7.98 | 17.8 | 5.57 |
| 1 | 5.19 | 6.55 | 6.80 | 8.48 | 16.8 | 6.10 |
| 2 | 4.23 | 6.08 | 6.21 | 5.87 | 7.74 | 4.59 |
| 3 | 3.74 | 5.54 | 5.67 | 5.20 | 8.45 | 5.03 |
| 4 | 3.34 | 5.51 | 6.37 | 5.29 | 4.86 | 4.48 |
| 6 | 3.64 | 4.65 | 7.11 | 4.76 | 5.73 | 3.91 |
| 8 | 3.21 | 4.09 | 5.84 | 4.09 | 5.33 | 3.31 |
| 24 | 1.64 | 2.53 | 3.53 | 2.21 | 1.84 | 1.96 |
| 48 | 1.35 | 1.43 | 1.83 | 1.19 | 0.70 | 1.11 |
| 72 | 0.88 | 0.97 | 1.07 | 0.89 | 0.76 | 0.69 |
| 96 | 0.73 | 0.88 | 0.83 | 0.84 | 0.53 | 0.60 |
| 120 | 0.61 | 0.81 | 0.61 | / | / | / |
| 168 | 0.68 | 0.74 | 0.45 | 0.62 | 0.27 | 0.50 |
| 192 | 0.59 | 0.73 | 0.24 | / | / | / |
| 216 | 0.55 | 0.77 | 0.16 | 0.48 | 0.10 | 0.43 |
| 240 | 0.40 | 0.62 | 0.10 | / | / | / |
| 336 | 0.53 | 0.82 | 0.12 | 0.19 | 0.07 | 0.35 |
| 432 | 0.43 | 0.52 | 0.13 | 0.12 | / | 0.31 |
| 504 | 0.39 | 0.27 | / | 0.10 | / | 0.31 |
| 600 | 0.27 | 0.12 | 0.03 | 0.09 | / | 0.32 |
| 720 | 0.21 | 0.19 | 0.05 | 0.08 | / | 0.32 |

### (3) Study on in vivo release characteristics of lumateperone-in situ gel preparations in rats

SD male rats (N=5) were selected to study the PK behavior *in vivo.* The dosage was 10.5 mg/kg (calculated as lumateperone). The preparations of some of the above Examples were injected subcutaneously. Blood was sampled from the tail vein at predetermined points in time. After the plasma samples were treated by Solid-Phase Extraction (SPE), the lumateperone concentration was determined by the LC-MS/MS method. The curve of plasma concentration vs. time (within 312 h) for the sample of Example 28 in rat was shown in FIG. 6.

**Table 6 Plasma Concentrations of Lumateerone-In Situ Gel in Rats**

| Time (h) | Plasma Concentrations (ng/mL) |
|---|---|
| | Example 28 |
| 0 | 0.00 |
| 0.25 | 28.6 |
| 0.5 | 44.9 |
| 0.75 | 46.8 |
| 1 | 49.5 |
| 2 | 48.6 |
| 2 | 50.8 |
| 3 | 39.4 |
| 4 | 38.6 |
| 6 | 26.7 |
| 8 | 19.2 |
| 24 | 4.32 |
| 48 | 1.93 |
| 72 | 1.34 |
| 168 | 1.40 |
| 192 | 1.46 |
| 216 | 1.56 |
| 264 | 2.27 |
| 312 | 2.22 |

In situ gels (active ingredients: rotigotine, pramipexole, lumateperone, etc.) prepared by the conventional Atrigel technology exhibited larger burst releases *in vitro* and were not suitable for drugs with narrow safety or therapeutic windows, but they exhibited a significant effect of slowing down the burst releases *in vivo* and *in vitro* after the addition of some hydrophobic additives (such as benzyl benzoate, glyceryl triacetate, glyceryl tricaprylate, and medium-chain glyceryl triacetate, etc.) and optionally hydrophilic gel matrices (such as poloxamer, sodium carboxymethylcellulose, carbomer, hydroxypropyl methyl cellulose, and polyvinylpyrrolidone, etc.), and the *in vivo* plasma concentration could be maintained in a safe and effect range for more than one week.

### Experimental Example 4: Study on rotigotine-related substances during storage

After being placed at 2 to 8°C under the dark condition for a specified time, 180 mg of the samples were weighed and placed in a 2-mL volumetric flask, to which 0.4 mL of stock solution of impurity as a reference substance was added, dissolved with a diluent and diluted to the scale. After shaking up, the content of the rotigotine-related substances was detected by HPLC. The detection results of the content of related substances in the samples of Example 15, Example 31, and Example 32 after stored for 0 day and 3 months were shown in Table 7.

**Table 7 Contents of Related Substances in Rotigotine-In Situ Gels Prepared by Different Methods During Storage**

| Example No. | | Example 15 | Example 15 | Example 31 | Example 31 | Example 32 | Example 32 |
|---|---|---|---|---|---|---|---|
| Storage Time | | 0 Day | 3 Months | 0 Day | 3 Months | 0 Day | 3 Months |
| Impurity B | Desthienylethyl rotigotine | 0.09% | 0.08% | 0.09% | 0.10% | 0.08% | 0.08% |
| Impurity C | Despropyl rotigotine | Not Detected | Not Detected | Not Detected | 0.17% | Not Detected | 0.18% |
| Impurity D | Ethyl-rotigotine | Not Detected | Not Detected | Not Detected | Not Detected | Not Detected | Not Detected |
| Impurity E | Rotigotine N-Oxide | Not Detected | Not Detected | Not Detected | 0.12% | Not Detected | 0.11% |
| Impurity F | Acetyl-rotigotine | Not Detected | Not Detected | Not Detected | Not Detected | Not Detected | Not Detected |
| Impurity K | 7,8-Dihydronaphthol | Not Detected | Not Detected | Not Detected | 0.12% | Not Detected | 0.11% |
| Impurity G | N,N-Dithienylethyl rotigotine | Not Detected | Not Detected | Not Detected | Not Detected | Not Detected | Not Detected |
| Impurity H | Rotigotine methyl ether | Not Detected | Not Detected | Not Detected | Not Detected | Not Detected | Not Detected |
| Impurity I | Rotigotine p-toluene sulfonate | Not Detected | Not Detected | Not Detected | Not Detected | Not Detected | Not Detected |
| Impurity J | Rotigotine thienylethyl ether | 0.15% | 0.15% | 0.16% | 0.19% | 0.16% | 0.17% |
| Other largest single impurities | | Not Detected | Not Detected | Not Detected | Not Detected | Not Detected | Not Detected |
| Total impurity content | | 0.24% | 0.23% | 0.25% | 0.70% | 0.24% | 0.65% |
| Total number of impurities | | 2 | 2 | 2 | 5 | 2 | 5 |

After the sample of Example 15 was stored at 2 to 8°C for 3 months under the dark condition, the types and content of the rotigotine-related substances did not change significantly. After stored for 0 day, the impurities in the samples of Examples 31 and 32 were similar to those in the sample of Example 15. After stored at 2 to 8°C for 3 months, the content of some of the active ingredient-related substances (impurities C, E, and K) slightly increased in the samples of Example 31 and Example 32, but they still met the quality requirements for the delivery preparations.

## Claims

1. A delivery preparation, comprising an active pharmaceutical ingredient and a gel carrier, wherein the gel carrier comprises a biodegradable polymer, an organic solvent, a hydrophobic additive, and optionally a hydrophilic gel matrix.

2. The delivery preparation according to claim 1, wherein the hydrophobic additive is one or more selected from the group consisting of ethyl acetate, medium-chain triglyceride, glyceryl triacetate, glyceryl tricaprylate, benzyl benzoate, and benzyl alcohol; preferably, the hydrophobic additive is one or more selected from the group consisting of benzyl benzoate, glyceryl triacetate, and glyceryl tricaprylate.

3. The delivery preparation according to claim 1 or 2, wherein the hydrophobic additive is 1% to 50% of the total mass of the gel carrier, preferably 5% to 30% of the total mass of the gel carrier.

4. The delivery preparation according to any one of claims 1 to 3, wherein the hydrophilic gel matrix is one or more selected from the group consisting of poloxamer, carbomer, polyvinylpyrrolidone, hydroxypropyl methyl cellulose, and sodium carboxymethyl cellulose; preferably, the hydrophilic gel matrix is one or more selected from the group consisting of poloxamer 188, carbomer, and polyvinylpyrrolidone.

5. The delivery preparation according to any one of claims 1 to 4, wherein the hydrophilic gel matrix, if present, is 0.5% to 15% of the total mass of the gel carrier, preferably 1% to 5% of the total mass of the gel carrier.

6. The delivery preparation according to any one of claims 1 to 5, wherein the biodegradable polymer is a polyester or a polyester copolymer; preferably, the biodegradable polymer is polylactide or lactide/glycolide copolymer; preferably, the biodegradable polymer is lactide/glycolide copolymer; more preferably, the molar ratio of lactide to glycolide in the lactide/glycolide copolymer is 50:50 to 95:5; and/or
the biodegradable polymer has a molecular weight of 5000 to 70000 Da; and/or
the biodegradable polymer is 20% to 50% of the total mass of the gel carrier.

7. The delivery preparation according to any one of claims 1 to 6, wherein the organic solvent is N-methyl pyrrolidone and/or dimethyl sulfoxide; and/or the mass ratio of the organic solvent to the hydrophobic additive is 1: 1 to 9:1.

8. The delivery preparation according to any one of claims 1 to 7, wherein the active pharmaceutical ingredient is 0.5% to 30% of the total mass of the delivery preparation; preferably, the active pharmaceutical ingredient is 3% to 20% of the total mass of the delivery preparation.

9. A preparation method for the delivery preparation according to any one of claims 1 to 8, comprising the following steps: weighing prescribed doses of a biodegradable polymer, an organic solvent, a hydrophobic additive, and optionally a hydrophilic gel matrix, and mixing to homogeneity, adding an active pharmaceutical ingredient, and mixing to homogeneity to obtain the delivery preparation.

10. The preparation method according to claim 9, wherein the active pharmaceutical ingredient is added just before administration of the delivery preparation.

11. The preparation method according to claim 9, wherein the active pharmaceutical ingredient is added immediately after the biodegradable polymer, the organic solvent, the hydrophobic additive, and optionally the hydrophilic gel matrix are mixed to homogeneity.

12. A preparation method for the delivery preparation according to any one of claims 1 to 8, comprising the following steps: weighing prescribed doses of a biodegradable polymer, an active pharmaceutical ingredient, an organic solvent, a hydrophobic additive, and optionally a hydrophilic gel matrix, and mixing to homogeneity to obtain the delivery preparation.
